# EUROPÄISCHE PATENTSCHRIFT

(11) **EP 4 226 952 B1**
(45) Veröffentlichungstag und Bekanntmachung des Hinweises auf die Patenterteilung: **26.03.2025**
(21) Anmeldenummer: 22187102.3
(22) Anmeldetag: 26.07.2022
(51) Int. Cl.: A61L 2/10, A61L 2/20, A61L 2/24, B08B 15/02

(54) **SCHLEUSENANORDNUNG ZUM TRANSFER VON ARTIKELN AUS EINEM GEBINDE IN DIE PROZESSKAMMER EINES CONTAINMENTS UNTER ASEPTISCHEN BEDINGUNGEN**
SLUICE ARRANGEMENT FOR TRANSFERRING ARTICLES FROM A CONTAINER INTO THE PROCESS CHAMBER OF A CONTAINER UNDER ASEPTIC CONDITIONS
DISPOSITIF SAS PERMETTANT DE TRANSFÉRER DES ARTICLES D'UN CONTENEUR DANS LA CHAMBRE DE TRAITEMENT D'UNE ENCEINTE DE CONFINEMENT DANS DES CONDITIONS ASEPTIQUES

(43) Veröffentlichungstag der Anmeldung: 16.08.2023
(73) Patentinhaber: SKAN AG, 4123 Allschwil (CH)
(72) Erfinder: Geiser, Christoph Joschi, 4313 Möhlin (CH); Landes, Robert, 79713 Bad Säckingen (DE); Zeller, Mike, 4246 Wahlen (CH)
(74) Vertreter: Maucher Jenkins Patent- und Rechtsanwälte

(56) Entgegenhaltungen:
- WO-A2-2020/016645
- CN-A- 102 821 858

## Beschreibung

### Anwendungsgebiet der Erfindung

Die vorliegende Erfindung betrifft eine in einem Aufstellraum positionierte Schleusenanordnung zum Transfer von Artikeln aus seriell zugeführten Gebinden in die von einem Gehäuse umgebene Prozesskammer eines Containments unter aseptischen Bedingungen. Der Prozesskammer ist eine Eingangsstation vorgelagert. Ein einzelnes Gebinde umfasst ein Behältnis mit einem aseptischen Innenraum und einem Durchlass, der mit einer Abdeckung verschlossen ist. Die im Innenraum gelagerten Artikel sollen nach dem Öffnen der Abdeckung in der Prozesskammer behandelt werden. Die Behältnisse der Gebinde haben die Gestalt wannenförmiger Tubs, und die Artikel sind z.B. pharmazeutische Phiolen, Vials oder Spritzen, die in systematisch angeordneten muldenförmigen Aufnahmekonturen von Nestern stecken.

### Stand der Technik

Aus der WO 2020/016 645 A2 ist eine Anordnung zum kontaminationsfreien Einschleusen eines sterilen Objektes aus einem Behältnis, welches mit einer semipermeablen Abdeckung verschlossen ist, in eine von einer Wandung umgebenen Arbeitskammer eines Containments bekannt. Die Anordnung umfasst zunächst eine Toreinheit mit einem in der Wandung angeordneten Zugangsflansch, der einen Durchgang von aussen in die Arbeitskammer ausbildet, und einer den Durchgang dicht verschliessenden Tür, die sich zur Offenstellung in die Arbeitskammer bewegen lässt. Zur Anordnung gehört ferner eine Behältnisaufnahme mit einem Auffang zur Halterung eines in die Behältnisaufnahme eingebrachten Behältnisses, einer Öffnung zum Einbringen des Behältnisses in den Auffang und einem Flansch zum Zusammenwirken mit dem Zugangsflansch.

Eine Dekontaminationseinheit ist dazu vorgesehen, bei geschlossener Tür, am Zugangsflansch angedockter Behältnisaufnahme und in der Behältnisaufnahme gelagertem Behältnis, eine der Tür zugewandte Aussenfläche der Abdeckung zu dekontaminieren. In Schliessstellung ist die Tür vonseiten der Arbeitskammer abgedichtet an den Zugangsflansch angefügt, und der Zugangsflansch umrandet einen Zwischenraum, der im Durchgang liegt. Die Abdeckung des in der Behältnisaufnahme eingestellten Behältnisses ist bei an den Zugangsflansch herangeschwenkter Behältnisaufnahme zwischen dem Flansch und dem Zugangsflansch abgedichtet. Die Dekontaminationseinheit ist mit Wirkungsrichtung in den Durchgang hinein direkt an der Tür oder seitlich der Toreinheit installiert, welche die Tür und den Zugangsflansch aufweist. Die Dekontaminationseinheit ist als Strahlungsquelle, z.B. UVC, UV, E-Beam oder Puls-Licht, oder als Begasungseinrichtung, z.B zur Verneblung einer H₂O₂-Lösung ausgebildet.

### Aufgabe der Erfindung

Angesichts der beschränkten Produktivität der gemäss vorbekanntem Stand der Technik existierenden Vorrichtungen und praktizierten Verfahren, liegt der Erfindung die Aufgabe zugrunde, eine für das hiesige Anwendungsgebiet effizientere Schleusenanordnung zum Transfer von Artikeln aus seriell zugeführten Gebinden in die Prozesskammer eines Containments unter aseptischen Bedingungen vorzuschlagen. Massgeblich hierbei sind ein möglichst hoher Automatisierungsgrad, die weitgehende Vermeidung manuellen Eingreifens und Gewährleistung hoher Reinheitserfordernisse. Ausserdem ist darauf zu orientieren, dass bei möglichst geringem zeitlichem und apparativem Dekontaminationsaufwand nur Artikel in die Prozesskammer transferiert werden, die zu verarbeiten sind.

### Übersicht über die Erfindung

Die erfindungsgemässe Schleusenanordnung ist zum Transfer von Artikeln unter aseptischen Bedingungen aus seriell zugeführten Gebinden in eine von einem Gehäuse umgebene Prozesskammer eines in einem Aufstellraum positionierten Containments konzipiert. Der Prozesskammer ist eine Eingangsstation vorgelagert. Ein einzelnes Gebinde umfasst:
- ein Behältnis mit einem aseptischen Innenraum und einem Durchlass, der mit einer Abdeckung verschlossen ist; und
- die im Innenraum gelagerten Artikel, die nach Öffnen der Abdeckung in der Prozesskammer zu behandeln sind.

Zum Transfer der Artikel aus den Gebinden in die Prozesskammer ist eine von der Eingangsstation in die Prozesskammer führende Passage vorgesehen, welche sich in einer zwischen Eingangsstation und Prozesskammer verlaufenden Trennwand als Ausschnitt befindet. In der Passage sitzt eine diese umrandende Profildichtung, welche zusammen mit einem in der Eingangsstation herangeführten, temporär angepressten Gebinde und zusammen mit einer temporär angepressten Dekontaminationsvorrichtung Abdichtungen schafft, wodurch die angrenzenden Oberflächen einen Rauminhalt umschliessen und sich dieser mit den angrenzenden Oberflächen mittels der Dekontaminationsvorrichtung dekontaminieren lässt.

Die Profildichtung besitzt:
- eine erste umlaufende Dichtfläche, die an der temporär angepressten Dekontaminationsvorrichtung mit einer ersten Kontaktlinie anliegt, welche eine Emissionsfläche an der Dekontaminationsvorrichtung abgrenzt; und
- eine zweite umlaufende Dichtfläche, die am temporär angepressten Gebinde mit einer zweiten Kontaktlinie anliegt, welche eine Prozessfläche auf der das Gebinde verschliessenden Abdeckung abgrenzt.

Die Emissionsfläche ist zumindest gleichgross oder grösser als die Prozessfläche.

Nachfolgend werden spezielle Ausführungsformen der Erfindung definiert:
In der Projektion ist die Prozessfläche mit der Emissionsfläche deckungsgleich oder die Prozessfläche ist in der grösseren Emissionsfläche vollständig eingebettet.

Wiederum in der Projektion betrachtet, ist die Emissionsfläche grösser als die Prozessfläche dimensioniert und hierbei überragt die Prozessfläche die Emissionsfläche partiell.

Die den Rauminhalt umschliessenden Oberflächen umfassen zumindest die Emissionsfläche, die Prozessfläche und die dem Rauminhalt zugewandten freiliegenden Flächen der Profildichtung. Zu den den Rauminhalt umschliessenden und zu dekontaminierenden Oberflächen kann auch ein Bereich der Stirnpartie von der Trennwand gehören.

Die Profildichtung weist eine dem Rauminhalt zugewandte Flankenkontur auf, entlang derer:
- an der ersten Dichtfläche die erste Kontaktlinie liegt, die zum Zusammenwirken mit der temporär angepressten Dekontaminationsvorrichtung bestimmt ist;
- an der zweiten Dichtfläche die zweite Kontaktlinie liegt, die zum Zusammenwirken mit der Abdeckung bestimmt ist, welche das temporär angepresste Gebinde verschliesst; und
- zwischen erster und zweiter Kontaktlinie Zwischenlinien definiert sind, wobei:
   zu einer durch den Querschnitt der Profildichtung gezogenen geometrischen Mittellinie die erste Kontaktlinie die grösste Spannweite aufweist, über die Zwischenlinien abwärts zur zweiten Kontaktlinie sich die Spannweiten bis hin zur zweiten Kontaktlinie mit der kleinsten Spannweite sukzessive verringern.

Die Profildichtung ist ein- oder mehrteilig an der in die Passage ragenden Stirnpartie der Trennwand angeordnet.

Die Dichtflächen sind an Enden lippenartiger Flügel ausgebildet, die sich gegenläufig von einem Basissteg erstrecken. Die Profildichtung weist ferner auf:
- zwei sich vom Basissteg erstreckende Schenkel, die zusammen mit dem Basissteg einen Kanal bilden, der zur Aufnahme der Stirnpartie der Trennwand bestimmt ist;
- jeweils eine Verjüngung am jeweiligen Übergang vom betreffenden Schenkel zum benachbarten Flügel, zwecks Begünstigung der Materialverformung und Dichteigenschaft beim Anpressen der Dekontaminationsvorrichtung bzw. des Gebindes auf die Profildichtung; und
- von den Schenkeln in den Kanal gerichtete Stege zum Form- und Kraftschluss mit in der Stirnpartie vorhandenen Nuten.

Die Dekontaminationsvorrichtung ist als Strahlungsquelle, z.B. UVC, UV, E-Beam oder Puls-Licht, ausgebildet. Die Profildichtung besteht aus einem Elastomer. Die Passage ist von rechteckiger oder rundlicher Gestalt.

Das Behältnis des Gebindes hat zuoberst einen umlaufenden Rand, auf dem die den Durchlass überspannende Abdeckung befestigt ist, z.B. durch Verkleben oder Verschweissen.

### Kurzbeschreibung der beigefügten Zeichnungen

Es zeigen:

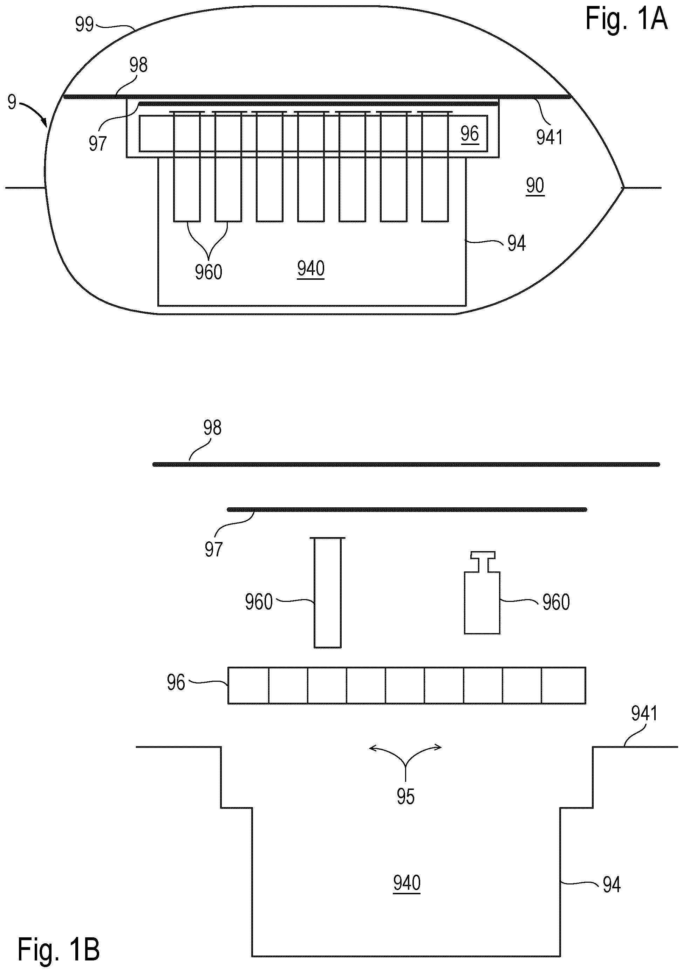

- Figur 1A -: ein mit der erfindungsgemässen Schleusenanordnung zu verarbeitendes Gebinde mit geschlossener Umhüllung;
- Figur 1B -: das Gebinde gemäss Figur 1A ohne die Umhüllung in Explosivansicht;

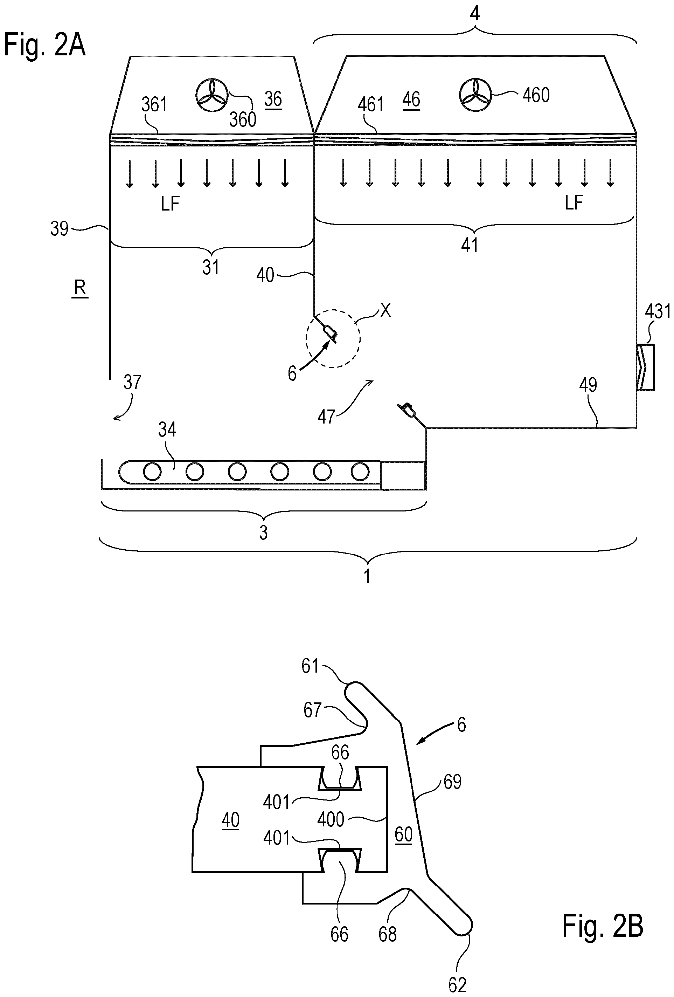

- Figur 2A -: die Schleusenanordnung in unvollkommenem Aufbau und ohne eingeführtes Gebinde, in Gesamtansicht;
- Figur 2B -: das vergrösserte Detail X aus Figur 2A, mit der in der Passage, an der Trennwand eingesetzten Dichtung und deren erste und zweite Dichtkontur unverpresst, im Querschnitt;

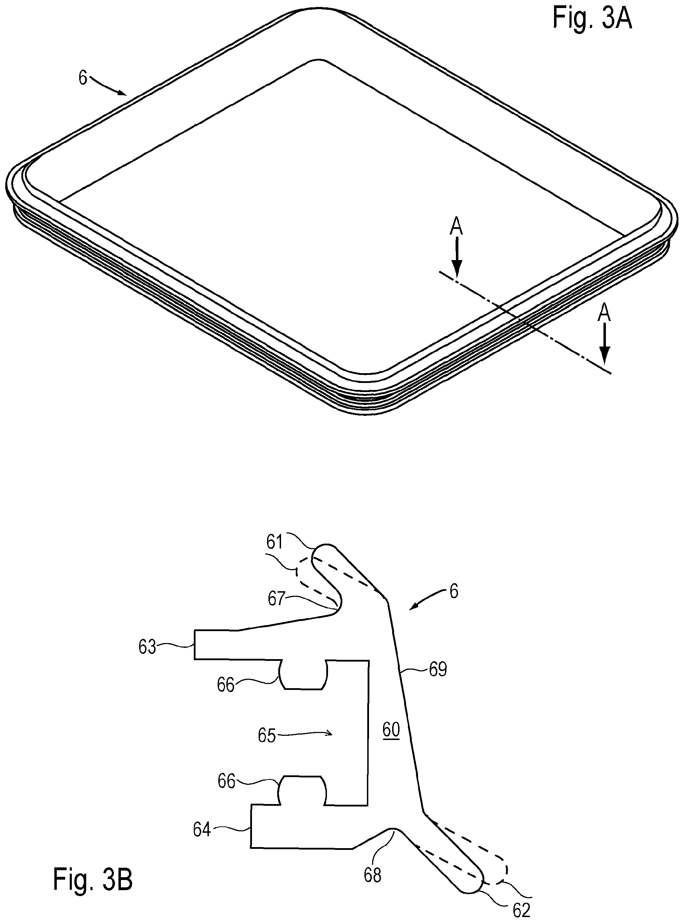

- Figur 3A -: die Profildichtung aus den Figuren 2A und 2B, in perspektivischer Draufsicht;
- Figur 3B -: den vergrösserten Schnitt A-A aus Figur 3A;

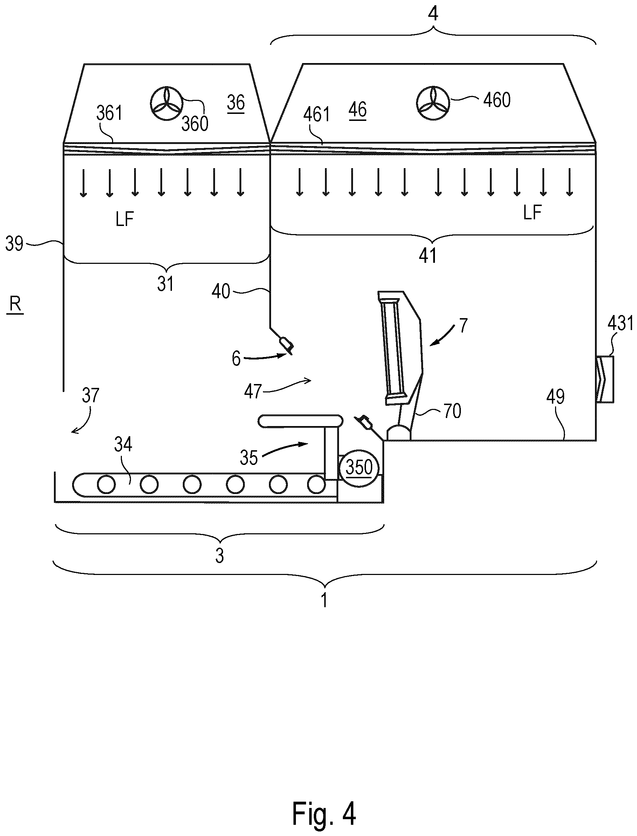

Figuren 4 bis 8: die Funktion der Profildichtung in verschiedenen Situationen, als Prinzipdarstellungen;
[Diese Situationen gibt es nicht alle im realen Prozessablauf und nicht alle in der Abfolge, sondern es sollen die verschiedenen Zustände an der Profildichtung im entspannten sowie im ein- und beidseitig beanspruchten Zustand illustriert werden.]
   - Figur 4 -: die Passage ist offen, die Dekontaminationsvorrichtung ist abgeschwenkt und noch kein Gebinde herangeführt;

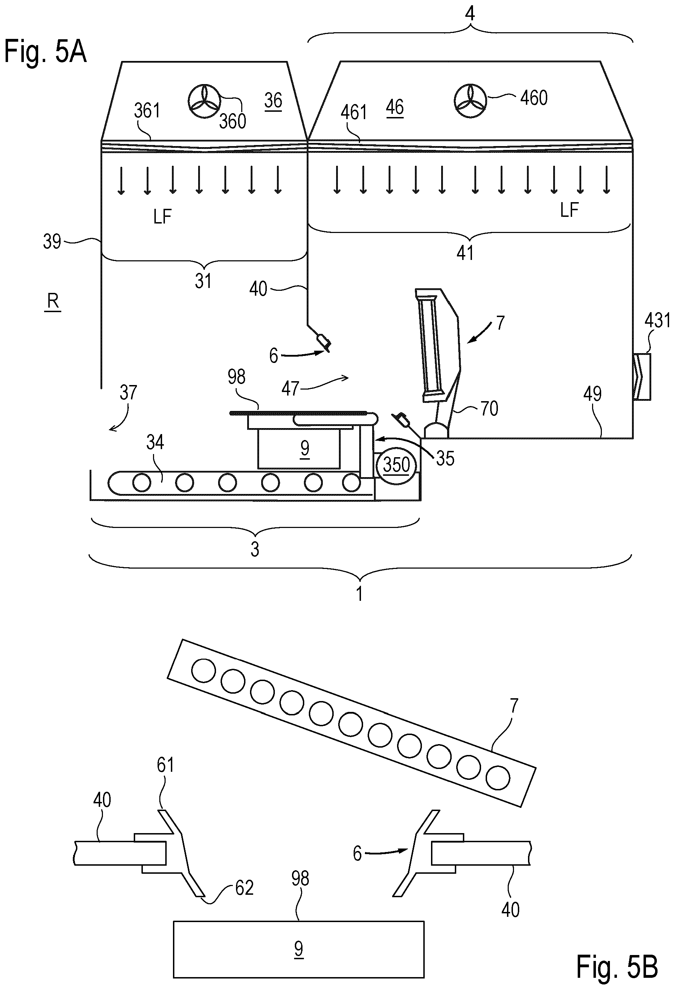

   - Figur 5A -: die Passage ist weiterhin offen und die Dekontaminationsvorrichtung abgeschwenkt; in der Eingangsstation ist die zweite Fördereinrichtung in der Ausgangsstellung mit einem Gebinde beladen;
   - Figur 5B -: die Situation an der Passage gemäss Figur 5A mit den Dichtflächen der Profildichtung somit in entspannter Position;

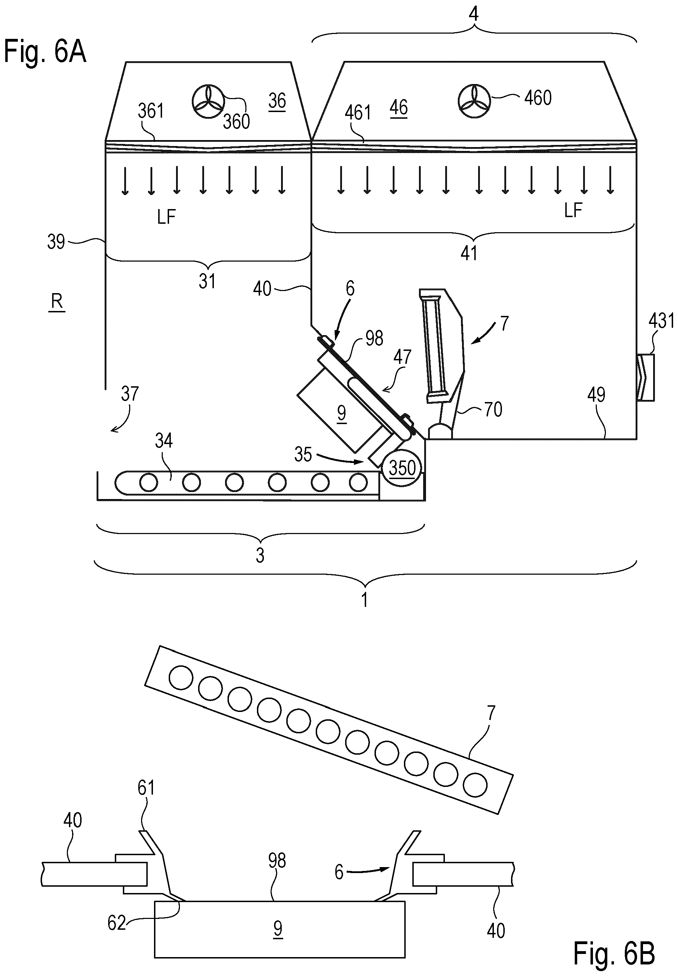

   - Figur 6A -: mittels der in die Hebestellung bewegten zweiten Fördereinrichtung ist ein Gebinde an die Passage herangeschwenkt, die Dekontami- nationsvorrichtung aber weiterhin abgeschwenkt;
   - Figur 6B -: die Situation an der Passage gemäss Figur 6A mit der zweiten Dichtfläche der Profildichtung somit in angepresster, deformierter Position;

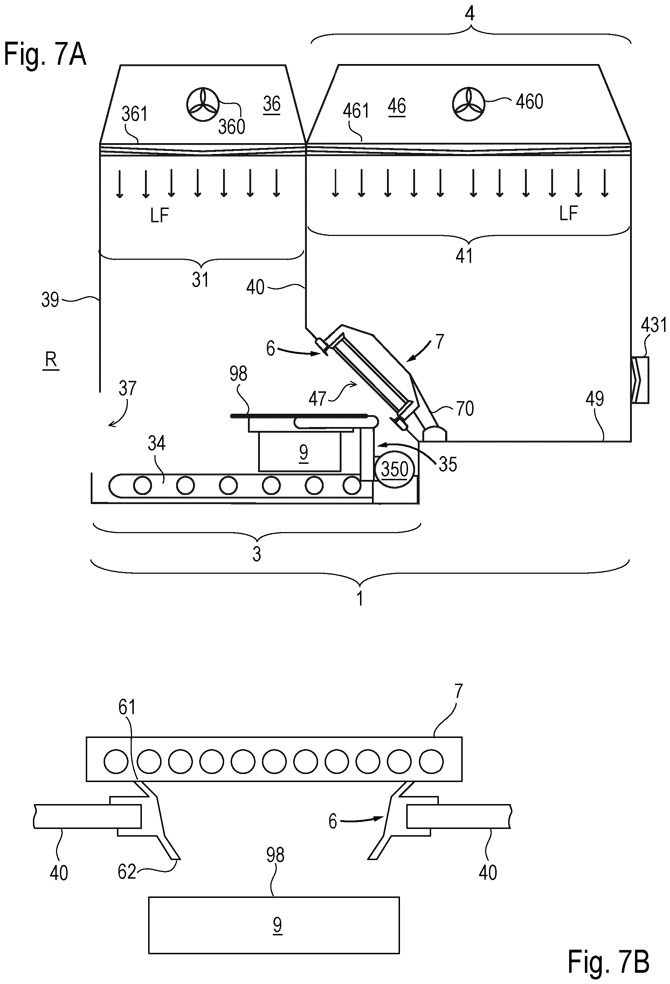

   - Figur 7A -: an die Passage ist die Dekontaminationsvorrichtung herangeschwenkt, nicht aber das Gebinde, mit dem die zweite Fördereinrichtung in der Ausgangsstellung beladen ist;
   - Figur 7B -: die Situation an der Passage gemäss Figur 7A mit der ersten Dichtfläche der Profildichtung somit in angepresster, deformierter Position;

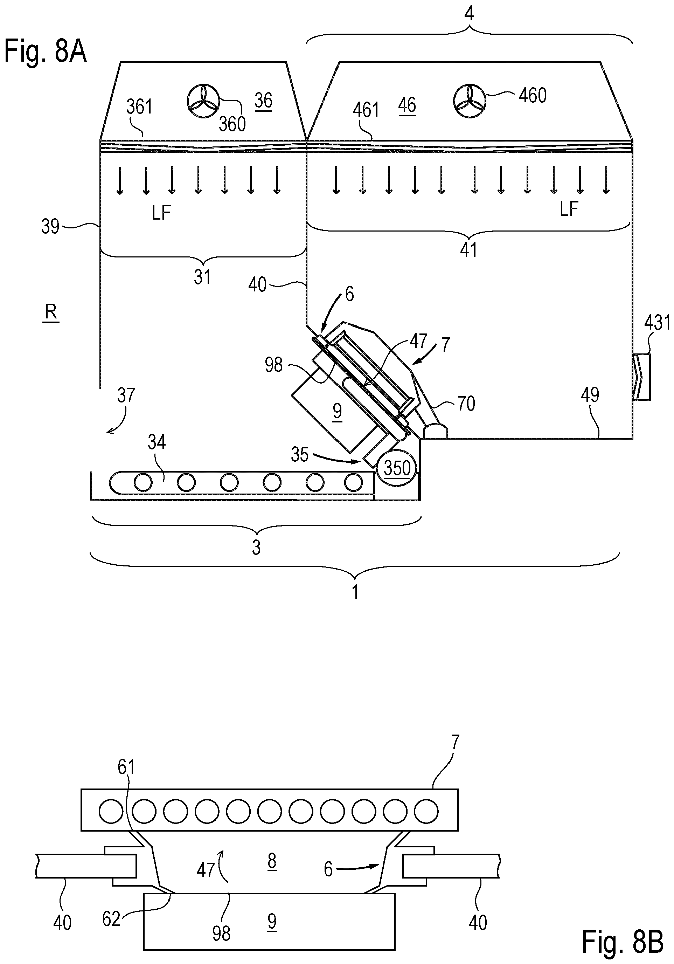

   - Figur 8A -: an die Passage sind die Dekontaminationsvorrichtung und mittels der in die Hebestellung bewegten zweiten Fördereinrichtung das Gebinde herangeschwenkt;
   - Figur 8B -: die Situation an der Passage gemäss Figur 8A mit der ersten und zweiten Dichtfläche der Profildichtung somit in angepresster, deformierter Position;

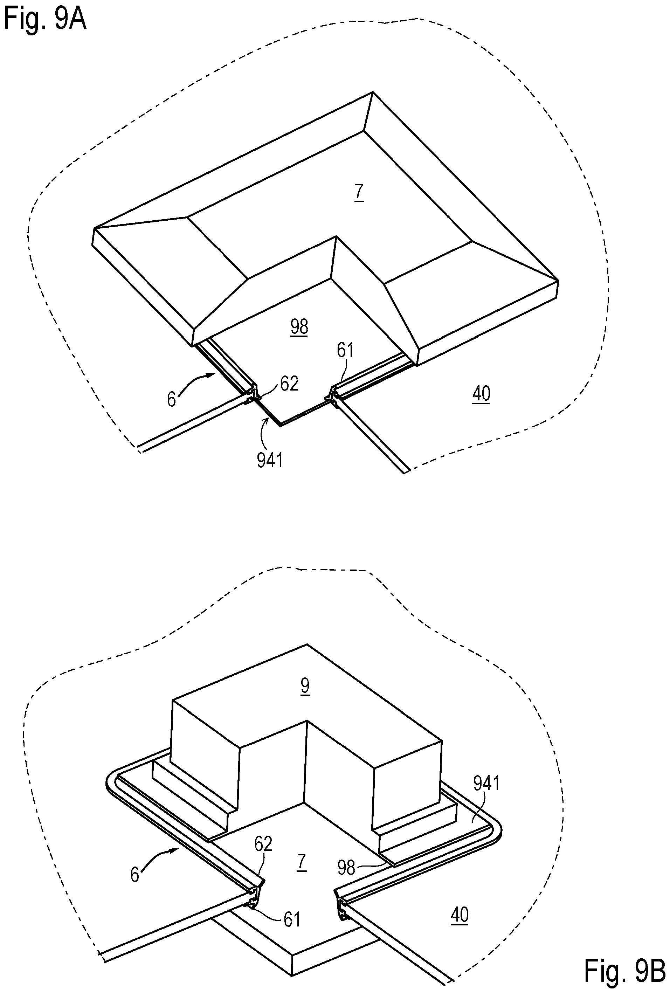

   - Figur 9A -: die Situation an der Passage gemäss Figuren 8A und 8B, in perspektivischer Sicht von oben, im Teilschnitt;
   - Figur 9B -: die Situation an der Passage gemäss Figuren 8A und 8B, in perspektivischer Sicht von unten, im Teilschnitt;

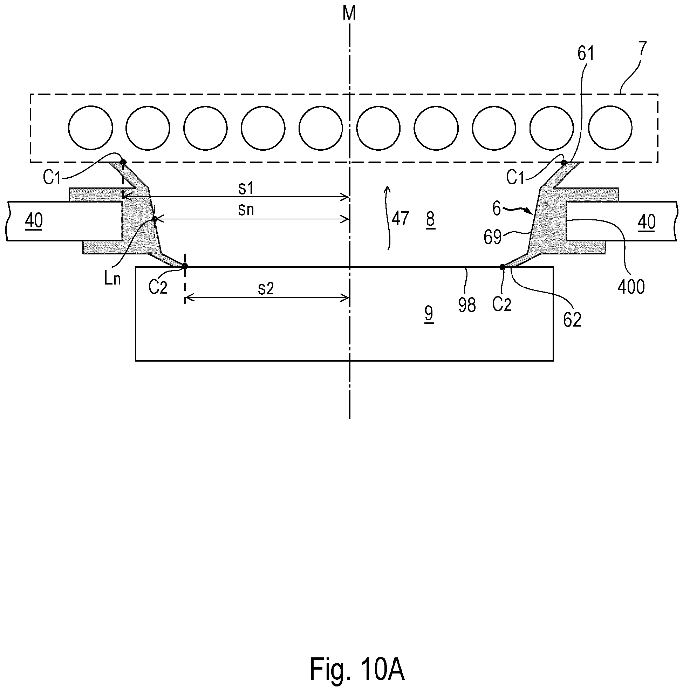

   - Figur 10A -: den Aufbau gemäss Figur 8B zur Veranschaulichtung der von der Profildichtung an deren Flankenkontur mit Dekontaminationsvorrichtung und Gebinde gebildeten Kontaktlinien und Spannweiten;

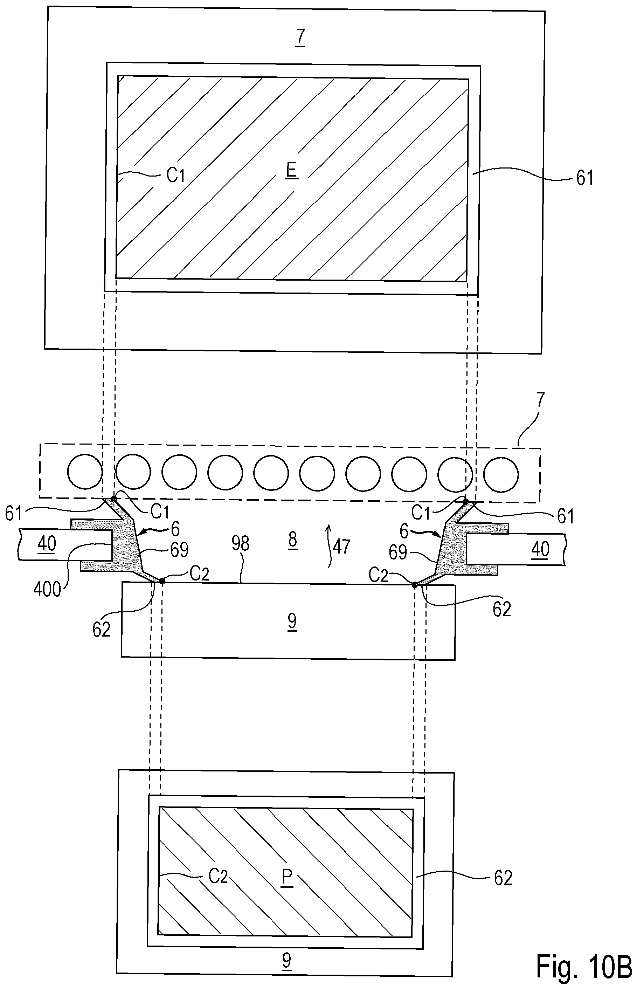

   - Figur 10B -: den Aufbau gemäss Figur 10A zur Veranschaulichtung der von den Kontaktlinien umrandeten Emissions- und Prozessflächen;

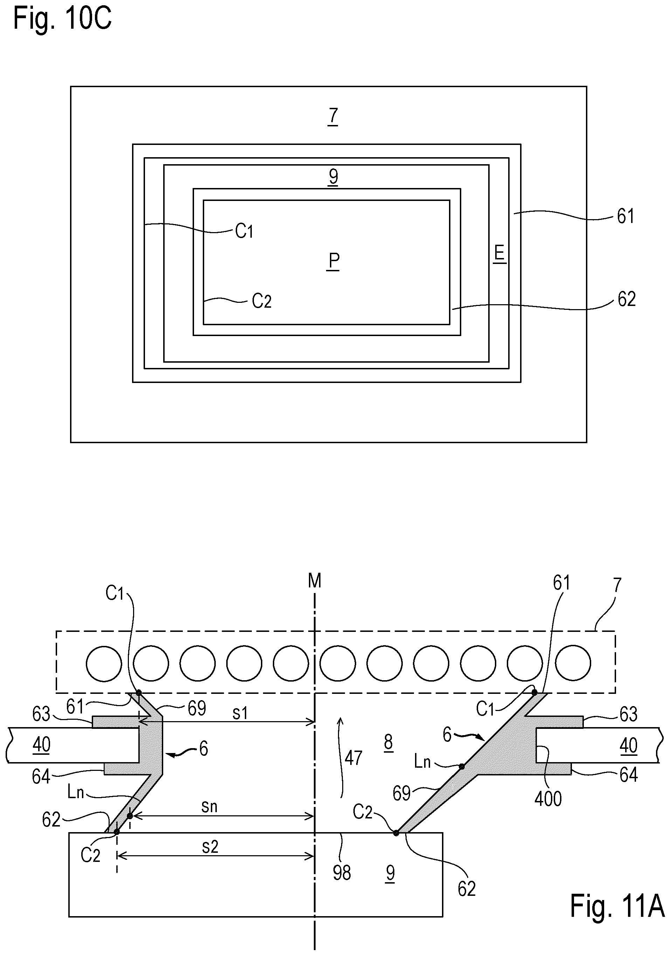

   - Figur 10C -: den Aufbau gemäss Figur 10B zur Veranschaulichtung der Überlagerung der entstandenen Emissions- und Prozessflächen;
   - Figur 11A -: den Aufbau gemäss Figur 10A mit einer im Querschnitt sich verändernden Profildichtung;

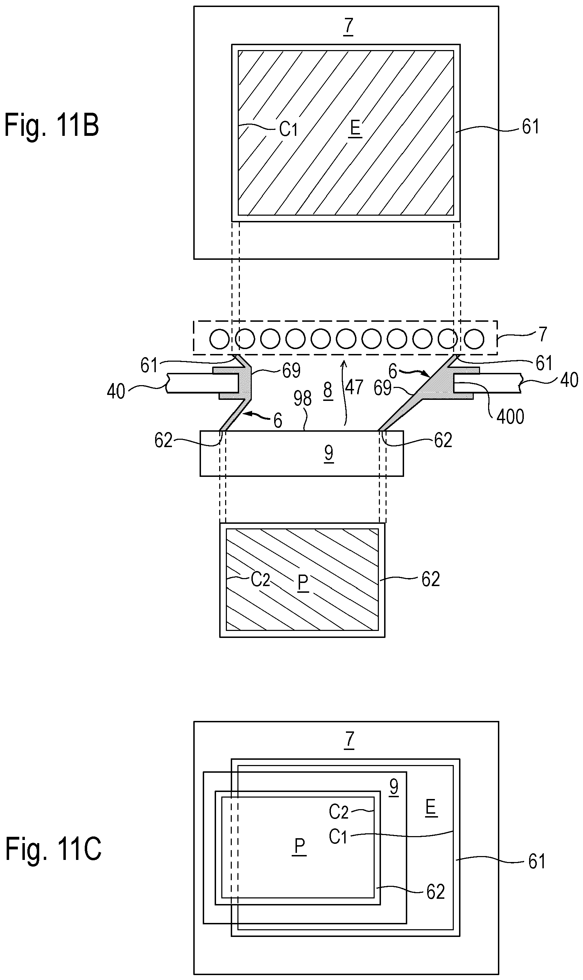

   - Figur 11B -: den Aufbau gemäss Figur 11A mit der sich im Querschnitt verändernden Profildichtung zur Veranschaulichtung der von den Kontaktlinien umrandeten Emissions- und Prozessflächen;
   - Figur 11C -: den Aufbau gemäss Figur 11B zur Veranschaulichtung der Überlagerung der entstandenen Emissions- und Prozessflächen;

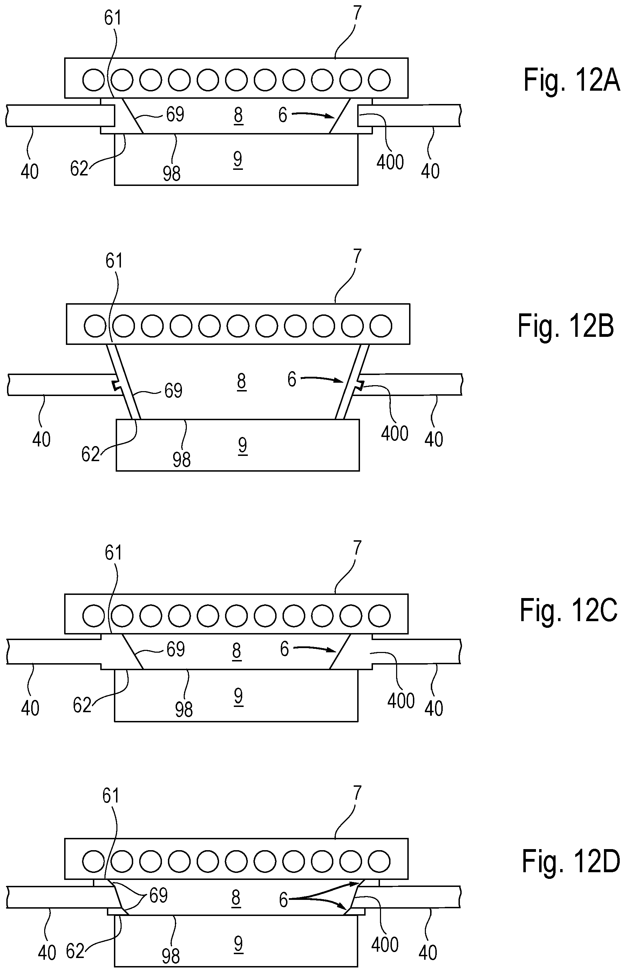

Figuren 12A bis 12D: vier alternative Formen der Profildichtung im Zustand mit an die Passage herangeschwenkter Dekontaminationsvorrichtung und Gebinde mittels der in die Hebestellung bewegten zweiten Fördereinrichtung, somit jeweils erste und zweite Dichtfläche der Profildichtung in angepresster, deformierter Position;
   - Figur 12A -: eine erste Alternativform der Profildichtung;
   - Figur 12B -: eine zweite Alternativform der Profildichtung;
   - Figur 12C -: eine dritte Alternativform der Profildichtung; und
   - Figur 12D -: eine vierte Alternativform der Profildichtung.

### Ausführungsbeispiel

Mit Bezug auf die beiliegenden Zeichnungen erfolgt nachstehend die detaillierte Beschreibung der erfindungsgemässen Schleusenanordnung zum Transfer von Artikeln aus seriell zugeführten Gebinden in die von einem Gehäuse umgebene Prozesskammer eines Containments unter aseptischen Bedingungen. Gemäss Darstellung in den Zeichnungsfiguren werden die Funktion der verwendeten Profildichtung, ihre alternativen Formen der sowie deren geometrische Verhältnisse erörtert.

Für die gesamte weitere Beschreibung gilt folgende Festlegung. Sind in einer Figur zum Zweck zeichnerischer Eindeutigkeit Bezugsziffern enthalten und dabei zeichnerisch eindeutig erkennbar ist, dass es sich um "wiederkehrende" Bauteile handelt, aber im unmittelbar zugehörigen Beschreibungstext nicht erläutert, so sei im Interesse der Verkürzung, auf deren Erklärung in vorangehenden Figurenbeschreibungen hingewiesen.

### Figuren 1A und 1B

Ein einzelnes Gebinde **9** umfasst:
- ein Behältnis **94** mit einem aseptischen Innenraum **940** und einem Durchlass **95,** der mit einer Abdeckung **98** verschlossen ist;
- ein im Innenraum **940** gelagertes Nest **96,** in dessen muldenförmigen Aufnahmekonturen die Artikel **960** stecken, welche nach Öffnen der Abdeckung **98** in der Prozesskammer **41** des Containments 4 zu behandeln sind;
- optional eine zwischen der Abdeckung **98** und dem Nest **96,** über den Artikeln **960** eingefügte Zwischenlage **97;** sowie
- eine im Anlieferungszustand das gesamte Gebinde 9 umgebende, geschlossene Umhüllung **99,** die das Innenvolumen **90** steril hält.

Das Behältnis **94** ist z.B. ein wannenförmiges Tub. Die Artikel **960** sind insbesondere Phiolen, Vials oder Spritzen. Von der Abdeckung **98** und der Umhüllung **99** bestehen vorzugsweise zumindest Flächenanteile aus einem semipermeablen Vliesgewebe, wie Tyvek^{®}. Die Abdeckung **98** ist typischerweise auf dem den Durchlass **95** des Behältnisses **94** umlaufenden Rand **941** versiegelt, z.B. verklebt oder verschweisst, und bewahrt so den Innenraum **940** des Behältnisses **94** in sterilem Zustand.

### Figuren 2A bis 3B

Die im Aufstellraum **R** installierte Schleusenanordnung **1** strukturiert sich in eine Eingangsstation **3** mit der Vorkammer **31** und das sich an die Eingangsstation **3** anschliessende Containment 4 mit dessen Prozesskammer **41.** Die Eingangsstation **3** ist vom Gehäuse **39** umgeben, in dem der in den Aufstellraum **R** mündende Zugang **37** vorgesehen ist. Innerlich der Vorkammer **31,** gleich hinter dem Zugang **37** beginnend, ist die erste Fördereinrichtung **34** installiert. Im Plenum **36** der Eingangsstation **3** sind ein Zuluftventilator **360** sowie ein Zuluftfilter **361** zur Erzeugung eines gereinigten, abwärts laminar strömenden Luftstromes **LF** angeordnet. Zwischen der Eingangsstation **3** und der Prozesskammer **41** erstreckt sich die Trennwand **40,** welche die Passage **47** aufweist. Auch im Plenum **46** des Containments **4** sitzen ein Zuluftventilator **460** und ein Zuluftfilter **461** zur Erzeugung eines gereinigten, abwärts laminar strömenden Luftstromes **LF.** Im die Prozesskammer **41** umgebenden Gehäuse **49** ist seitlich und/oder alternativ in dessen Boden ein Abluftfilter **431** vorgesehen.

In der Passage **47,** welche vorzugsweise von rechteckiger oder rundlicher Gestalt ist, sitzt die äquivalent geformte Profildichtung **6,** nämlich an der in die Passage **47** ragenden Stirnpartie **400** der Trennwand **40.** Als Material für die Profildichtung **6** eignet besonders z.B. ein Elastomer. An der Profildichtung **6** sind eine erste Dichtfläche **61** und eine zweite Dichtfläche **62** an Enden lippenartiger Flügel ausgebildet, die sich gegenläufig von einem Basissteg **60** erstrecken und zusammen eine in die Passage **47** weisende Flankenkontur **69** ergeben. Die Profildichtung **6** weist ferner zwei sich vom Basissteg **60** erstreckende Schenkel **63,64** auf, die zusammen mit dem Basissteg **60** einen Kanal **65** bilden, der zur Aufnahme der Stirnpartie **400** der Trennwand **40** bestimmt ist. Am jeweiligen Übergang vom betreffenden Schenkel **63,64** zum benachbarten Flügel ist jeweils eine Verjüngung **67,68** vorgesehen, welche die Materialverformung und Dichteigenschaft beim Anpressen der Dekontaminationsvorrichtung 7 bzw. des Gebindes 9 auf die Profildichtung 6 begünstigt (siehe ab Figur 6A). Von den Schenkeln **63,64** erstrecken sich in den Kanal **65** gerichtete Stege **66** zwecks Form- und Kraftschluss mit in der Stirnpartie **400** vorhandenen Nuten **401.**

### Figuren 4 bis 5B

Diese Situationen finden sich nicht im realen Prozessablauf, sondern sollen nur die offene Passage und damit die momentan funktionslose Profildichtung **6** im entspannten Zustand illustrieren.

Zum Transport der Gebinde **9** schliesst sich an die erste Fördereinrichtung **34** die zweite Fördereinrichtung **35** an, die um eine Achse **350** aufwärts in Richtung Passage **47** schwenkbar ist. In der Prozesskammer **41** ist ein schwenkbarer Ausleger **70** verankert, an dem die Dekontaminationsvorrichtung **7** installiert ist, um diese im Arbeitsrhythmus der Schleusenanordnung **1** an die Passage **47** heranzuführen und wieder abzuheben. Gegenwärtig ist die Dekontaminationsvorrichtung **7** von der Passage **47** abgeschwenkt und noch kein Gebinde **9** herangeführt (s. Figur 4).

In der Eingangsstation **3** ist das aus der Umhüllung **99** ausgebrachte und durch den Zugang **37** auf die erste Fördereinrichtung **34** geladene Gebinde **9** von der zweiten Fördereinrichtung **35** übernommen. Das Gebinde **9** ist noch nicht an der Passage **47** angedockt, und die zuoberst des Gebindes **9** dieses verschliessende Abdeckung **98** bleibt zunächst unbehandelt. Die Dekontaminationsvorrichtung **7** ist weiterhin von der Passage **47** abgehoben. Bei beidseits offener Passage **47** wären die Dichtflächen **61,62** der Profildichtung **6** somit ohne Kontakt mit dem Gebinde **9** und der Dekontaminationsvorrichtung **7,** daher im entspannten, funktionslosen Zustand (Figuren 5A und 5B).

### Figuren 6A und 6B

Von der zweiten Fördereinrichtung **35** wurde das Gebinde **9** an die Passage **47** herangeführt, welche dadurch vonseiten der Vorkammer **31** verschlossen ist, während die Dekontaminationsvorrichtung **7** weiterhin in abgeschwenkter Position steht. Die zweite Dichtfläche **62** der Profildichtung **6** sitzt somit in angepresstem, deformiertem Zustand auf der Abdeckung **98** auf, hingegen bleibt die erste Dichtfläche **61,** welche zur Dekontaminationsvorrichtung **7** orientiert ist, unbelastet. Das Heranführen der Gebinde **9** an die Passage **47** und/oder an die zweite Fördereinrichtung **35** kann insbesondere durch Roboter realisiert sein.

### Figuren 7A und 7B

Quasi in Umkehrung zur vorherigen Situation ist nun die Dekontaminationsvorrichtung **7** auf die Passage **47** geschwenkt. Das Gebinde **9** ist auf der zweiten Fördereinrichtung **35** geladen und steht zugleich auf der ersten Fördereinrichtung **34.** Somit ist die Passage **47** jetzt vonseiten der Prozesskammer **41** verschlossen. Die Dekontaminationsvorrichtung **7** drückt deformierend auf die erste Dichtfläche **61** der Profildichtung **6,** während die zweite, zum Gebinde **9** orientierte Dichtfläche **62** unbelastet ist.

### Figuren 8A bis 9B

Vonseiten der Vorkammer **31** sind das Gebinde **9** mittels der zweiten Fördereinrichtung **35** und zugleich mit dem geschwenkten Ausleger **70** die Dekontaminationsvorrichtung **7** an die Passage **47** herangeführt, welche damit beidseits verschlossen ist. Somit kommen die erste Dichtfläche **61** von der aufsitzenden Dekontaminationsvorrichtung **7** und zugleich die zweite Dichtfläche **62** vom angedockten Gebinde **9** mit der Abdeckung **98** infolge Anpressens in Funktion. In diesem Status kann die Dekontaminationsvorrichtung **7** aktiviert werden, um die zugewandte Oberfläche der Abdeckung **98** sowie den eingeschlossenen Rauminhalt **8** mit den angrenzenden Oberflächen für den weiteren Prozessablauf dekontaminierend zu behandeln. Die Dekontaminationsvorrichtung **7** ist als Strahlungsquelle, z.B. UVC, UV, E-Beam oder Puls-Licht, ausgebildet. Es folgen das Abschwenken der Dekontaminationsvorrichtung **7** von Passage **47,** Öffnen der Abdeckung **98** und schliesslich der Transfer der Artikel **960** aus dem Innenraum **940** des Behältnisses **94** des Gebindes **9** in die Prozesskammer **41** unter aseptischen Bedingungen.

### Figuren 10A bis 10C

Diese Figurenfolge veranschaulicht mit Bezug auf Figur 8B die:
- von der Profildichtung **6** an deren Flankenkontur **69** zusammen mit der Dekontaminationsvorrichtung **7** und Gebinde **9** gebildeten Kontaktlinien **C1,C2** und Zwischenlinie **Ln** sowie Spannweiten **s1,s2,sn;**
- von den Kontaktlinien **C1,C2** umrandete Emissionsfläche **E** und Prozessfläche **P;** und
- Überlagerung der entstandenen Emissionsfläche **E** und Prozessfläche **P.**

Die Profildichtung **6** weist eine dem Rauminhalt **8** zugewandte Flankenkontur **69** auf, entlang derer:
- an der ersten Dichtfläche **61** die erste Kontaktlinie **C1** liegt, die zum Zusammenwirken mit der temporär angepressten Dekontaminationsvorrichtung **7** bestimmt ist;
- an der zweiten Dichtfläche **62** die zweite Kontaktlinie **C2** liegt, die zum Zusammenwirken mit der Abdeckung **98** bestimmt ist, welche das temporär angepresste Gebinde **9** verschliesst; und
- zwischen erster und zweiter Kontaktlinie **C1,C2** Zwischenlinien **Ln** definiert sind.

Zu einer durch den Querschnitt der Profildichtung **6** gezogenen geometrischen Mittellinie **M** hat die erste Kontaktlinie **C1** die grösste Spannweite **s1.** Über die Zwischenlinien **Ln** abwärts zur zweiten Kontaktlinie **C2,** verringern sich die Spannweiten **sn** sukzessive bis hin zur zweiten Kontaktlinie **C2** mit der kleinsten Spannweite **s2.**

Die erste Kontaktlinie **C1** umrandet eine Emissionsfläche **E** an der Dekontaminationsvorrichtung **7;** und die zweite Kontaktlinie **C2** umrandet eine Prozessfläche **P** auf der das Gebinde **9** verschliessenden Abdeckung **98.** Auf jeden Fall ist die Emissionsfläche **E** zumindest gleichgross oder grösser als die Prozessfläche **P** zu bemessen. Im hiesigen Beispiel ist die Emissionsfläche **E** deutlich grösser als die Prozessfläche **P** dimensioniert und umbettet hierbei in der Projektion die Prozessfläche **P** allseits. Damit ist sichergestellt, dass die aus der Dekontaminationsvorrichtung **7** austretende Strahlung eine ordnungsgemässe Dekontamination des Rauminhalts **8** und aller diesen umschliessenden Oberflächen bewirkt, nämlich das den Rauminhalt **8** füllende Gasvolumen, die Prozessfläche **P** auf der das Gebinde **9** verschliessenden Abdeckung **98** und die dem Rauminhalt **8** zugewandten freiliegenden Flächen der Profildichtung **6.** Keine der zu dekontaminierenden Oberflächen oder Raumanteile liegen im Schatten der Strahlung.

### Figuren 11A bis 11C

Der in dieser Figurenfolge dargestellte Aufbau weist gegenüber der vorherigen Figurenfolge 10A-10C folgende Unterschiede auf:
- Die Profildichtung **6** ist im Querschnitt anders gestaltet, der überdies nicht durchgehend gleich ist, sondern sich in dessen Verlauf verändert, wie in Figur 11A auf der linken Seite im Vergleich zur rechten Seite ersichtlich. Eine solche Gestaltung kann sich ergeben, wenn zwei verschiedene Hälften einer Profildichtung **6** zusammengefügt sind oder bei der Herstellung für eine einteilige Profildichtung **6** eine entsprechende Form dient.
- Entlang der Flankenkontur **69,** wiederum bezogen auf die geometrische Mittellinie **M,** erfüllt der in Figur 11A, rechts, dargestellte Querschnitt der Profildichtung **6** die Merkmale, dass die erste Kontaktlinie **C1** die grösste Spannweite **s1** hat und über die Zwischenlinien **Ln** sich abwärts zur zweiten Kontaktlinie **C2** die Spannweiten **sn** sukzessive verringern, bis hin zur zweiten Kontaktlinie **C2** mit der kleinsten Spannweite **s2.**

Anders der in Figur 11A, links, dargestellte Querschnitt der Profildichtung **6.** Unterhalb des unteren Schenkels **64** beginnen sich hier die Spannweiten **sn** der Zwischenlinien **Ln** sukzessive zu vergrössern, bis hin zur zweiten Kontaktlinie **C2** mit der grössten Spannweite **s2.** Auch bei diesem kombinierten Querschnitt der Profildichtung **6** ist folglich die Emissionsfläche **E** grösser als die Prozessfläche **P** dimensioniert. Da in der Projektion nun die Prozessfläche **P** die Emissionsfläche **E** einseitig überragt, wird die Prozessfläche **P** nur mehr dreiseitig von der Emissionsfläche **E** umgeben.

Massgeblich ist wiederum, dass die Emissionsfläche **E** zumindest gleichgross oder grösser als die Prozessfläche **P** ist und keine der zu dekontaminierenden Oberflächen oder Raumanteile im Schatten der dekontaminierenden Strahlung liegen.

### Figuren 12A bis 12D

Mit dieser Figurenfolge werden vier alternative Formen der Profildichtung **6** vorgeschlagen, die sich im Zustand mit an die Passage **47** herangeführter Dekontaminationsvorrichtung **7** und angedocktem Gebinde 9 befinden, so dass die jeweiligen ersten Dichtflächen **61** und jeweiligen zweiten Dichtflächen **62** der Profildichtung **6** angepresst und deformiert sind.

Bei der ersten Alternativform ist die Profildichtung **6** einteilig beschaffen und auf der Stirnpartie **400** der Trennwand **40** angebracht. Die Flankenkontur **69** ist keilförmig ausgebildet (Figur 12A). Bei der zweiten Alternativform ist die Profildichtung 6 ebenfalls einteilig beschaffen und auf der Stirnpartie **400** der Trennwand **40** angebracht. Die Flankenkontur **69** entsteht durch die schräg stehende plattenförmige Profildichtung **6** (Figur 12B). Die dritte Alternativform der Profildichtung **6** setzt sich einstückig von der Stirnpartie **400** fort, und die Flankenkontur **69** ist keilförmig ausgebildet (Figur 12C).

Die vierte Alternativform der Profildichtung **6** ist zweiteilig, wobei sich die erste Dichtfläche **61** am oberen Dichtungsteil befindet, das oben auf der Stirnpartie **400** fixiert ist, während die zweite Dichtfläche **62** am oberen Dichtungsteil liegt, das unterhalb der Stirnpartie **400** sitzt. Die dem Rauminhalt **8** zugewandte Frontfläche der Stirnpartie **400** bildet den Mittelteil der Flankenkontur **69** (Figur 12D).

Bei allen vier Formen sind die Merkmale erfüllt, wonach, auf die geometrische Mittellinie **M** bezogen, die erste Kontaktlinie **C1** die grösste Spannweite **s1** hat und über die Zwischenlinien **Ln** sich abwärts zur zweiten Kontaktlinie **C2** die Spannweiten **sn** sukzessive verringern, bis hin zur zweiten Kontaktlinie **C2** mit der kleinsten Spannweite **s2.**

Für die Funktion der Schleusenanordnung **1** ist auch hier massgeblich, dass die an der Dekontaminationsvorrichtung **7** mit der jeweils anliegenden Profildichtung **6** erzeugte Emissionsfläche **E** nicht kleiner ist als die Prozessfläche **P,** welche auf der Oberseite der das Gebinde **9** verschliessenden Abdeckung **98** gebildet wird.

## Patentansprüche

1. Schleusenanordnung (1) zum Transfer von Artikeln (960) unter aseptischen Bedingungen aus seriell zugeführten Gebinden (9) in eine von einem Gehäuse (49) umgebene Prozesskammer (41) eines in einem Aufstellraum (R) positionierten Containments (4), wobei:
a) der Prozesskammer (41) eine Eingangsstation (3) vorgelagert ist;
b) ein einzelnes Gebinde (9) umfasst:
ba) ein Behältnis (94) mit einem aseptischen Innenraum (940) und einem Durchlass (95), der mit einer Abdeckung (98) verschlossen ist; und
bb) die im Innenraum (940) gelagerten Artikel (960), die nach Öffnen der Abdeckung (98) in der Prozesskammer (41) zu behandeln sind;
c) zum Transfer der Artikel (960) aus den Gebinden (9) in die Prozesskammer (41) eine von der Eingangsstation (3) in die Prozesskammer (41) führende Passage (47) vorgesehen ist, welche sich in einer zwischen Eingangsstation (3) und Prozesskammer (41) verlaufenden Trennwand (40) als Ausschnitt befindet;
d) in der Passage (47) eine diese umrandende Profildichtung (6) sitzt, welche zusammen mit einem in der Eingangsstation (3) herangeführten, temporär angepressten Gebinde (9) und zusammen mit einer temporär angepressten Dekontaminationsvorrichtung (7) Abdichtungen schafft, wodurch die angrenzenden Oberflächen einen Rauminhalt (8) umschließen und sich dieser mit den angrenzenden Oberflächen mittels der Dekontaminationsvorrichtung (7) dekontaminieren lässt, wobei
e) die Profildichtung (6) besitzt:
ea) eine erste umlaufende Dichtfläche (61), die an der temporär angepressten Dekontaminationsvorrichtung (7) mit einer ersten Kontaktlinie (Cl) anliegt, welche eine Emissionsfläche (E) an der Dekontaminationsvorrichtung (7) abgrenzt; und
eb) eine zweite umlaufende Dichtfläche (62), die am temporär angepressten Gebinde (9) mit einer zweiten Kontaktlinie (C2) anliegt, welche eine Prozessfläche (P) auf der das Gebinde (9) verschließenden Abdeckung (98) abgrenzt; und
f) die Emissionsfläche (E) zumindest gleich groß oder größer als die Prozessfläche (P) ist, und
dass die Profildichtung (6) eine dem Rauminhalt (8) zugewandte Flankenkontur (69) aufweist, entlang derer:
g) an der ersten Dichtfläche (61) die erste Kontaktlinie (Cl) liegt, die zum Zusammenwirken mit der temporär angepressten Dekontaminationsvorrichtung (7) bestimmt ist;
h) an der zweiten Dichtfläche (62) die zweite Kontaktlinie (C2) liegt, die zum Zusammenwirken mit der Abdeckung (98) bestimmt ist, welche das temporär angepresste Gebinde (9) verschließt; und
i) zwischen erster und zweiter Kontaktlinie (Cl, C2) Zwischenlinien (Ln) definiert sind, wobei:
j) zu einer durch den Querschnitt der Profildichtung (6) gezogenen geometrischen Mittellinie (M) die erste Kontaktlinie (Cl) die größte Spannweite (sl) aufweist, über die Zwischenlinien (Ln) abwärts zur zweiten Kontaktlinie (C2) sich die Spannweiten (sn) bis hin zur zweiten Kontaktlinie (C2) mit der kleinsten Spannweite (s2) sukzessive verringern.

2. Schleusenanordnung (1) nach Anspruch 1, **dadurch gekennzeichnet, dass** in der Projektion die Prozessfläche (P) mit der Emissionsfläche (E) deckungsgleich ist oder die Prozessfläche (P) in der größeren Emissionsfläche (E) vollständig eingebettet ist.

3. Schleusenanordnung (1) nach Anspruch 1, **dadurch gekennzeichnet, dass** in der Projektion die Emissionsfläche (E) grösser als die Prozessfläche (P) dimensioniert ist und hierbei die Prozessfläche (P) die Emissionsfläche (E) partiell überragt.

4. Schleusenanordnung (1) nach zumindest einem der Ansprüche 1 bis 3, **dadurch gekennzeichnet, dass** die den Rauminhalt (8) umschließenden Oberflächen zumindest die Emissionsfläche (E), die Prozessfläche (P) und die dem Rauminhalt (8) zugewandten freiliegenden Flächen der Profildichtung (6) umfassen.

5. Schleusenanordnung (1) nach zumindest einem der Ansprüche 1 bis 4, **dadurch gekennzeichnet, dass** zu den den Rauminhalt (8) umschließenden und zu dekontaminierenden Oberflächen auch ein Bereich der Stirnpartie (400) von der Trennwand (40) gehören kann.

6. Schleusenanordnung (1) nach zumindest einem der Ansprüche 1 bis 5, **dadurch gekennzeichnet, dass** die Profildichtung (6) ein- oder mehrteilig an der in die Passage (47) ragenden Stirnpartie (400) der Trennwand (40) angeordnet ist.

7. Schleusenanordnung (1) nach zumindest einem der Ansprüche 1 bis 6, **dadurch gekennzeichnet, dass** die Dichtflächen (61, 62) an Enden lippenartiger Flügel ausgebildet sind, die sich gegenläufig von einem Basissteg (60) erstrecken.

8. Schleusenanordnung (1) nach Anspruch 7, **dadurch gekennzeichnet, dass** die Profildichtung (6) ferner aufweist:
a) zwei sich vom Basissteg (60) erstreckende Schenkel (63,64), die zusammen mit dem Basissteg (60) einen Kanal (65) bilden, der zur Aufnahme der Stirnpartie (400) der Trennwand (40) bestimmt ist;
b) jeweils eine Verjüngung (67,68) am jeweiligen Übergang vom betreffenden Schenkel (63, 64) zum benachbarten Flügel, zwecks Begünstigung der Materialverformung und Dichteigenschaft beim Anpressen der Dekontaminationsvorrichtung (7) bzw. des Gebindes (9) auf die Profildichtung (6); und
c) von den Schenkeln (63,64) in den Kanal (65) gerichtete Stege (66) zum Form- und Kraftschluss mit in der Stirnpartie (400) vorhandenen Nuten (401).

9. Schleusenanordnung (1) nach zumindest einem der Ansprüche 1 bis 8, **dadurch gekennzeichnet, dass** die Dekontaminationsvorrichtung (7) als Strahlungsquelle, z.B. UVC, UV, E-Beam oder Puls-Licht, ausgebildet ist.

10. Schleusenanordnung (1) nach zumindest einem der Ansprüche 1 bis 9, **dadurch gekennzeichnet, dass** die Profildichtung (6) aus einem Elastomer besteht.

11. Schleusenanordnung (1) nach zumindest einem der Ansprüche 1 bis 10, **dadurch gekennzeichnet, dass** die Passage (47) von rechteckiger oder rundlicher Gestalt ist.

12. Schleusenanordnung (1) nach zumindest einem der Ansprüche 1 bis 11, **dadurch gekennzeichnet, dass** das Behältnis (94) des Gebindes (9) zuoberst einen umlaufenden Rand (941) hat, auf dem die den Durchlass (95) überspannende Abdeckung (98) befestigt ist, z.B. durch Verkleben oder Verschweißen.

## Claims

1. Sluice arrangement (1) for transferring articles (960) under aseptic conditions from serially supplied packages (9) into a process chamber (41) surrounded by a housing (49) of a containment (4) positioned in an installation room (R), wherein:
a) the process chamber (41) is preceded by an input station (3);
b) an individual package (9) comprises:
ba) a container (94) having an aseptic interior (940) and a passage (95) that is closed by a cover (98); and
bb) the articles (960) stored in the interior (940) that are to be treated in the process chamber (41) after the cover (98) has been opened;
c) a passage (47) leading from the input station (3) into the process chamber (41) is provided for transferring the articles (960) from the packages (9) into the process chamber (41), which passage (47) is located in a partition wall (40) extending between the input station (3) and the process chamber (41) as a cutout;
d) a profile seal (6) is seated in the passage (47), which surrounds the latter and, together with a package (9) that is introduced and temporarily pressed into the input station (3), and together with a decontamination device (7) that is temporarily pressed into place, creates seals, whereby the adjacent surfaces enclose a spatial content (8) and the latter can be decontaminated with the adjacent surfaces by means of the decontamination device (7), wherein
e) e) the profile seal (6) comprises:
ea) a first circumferential sealing surface (61), which rests against the temporarily pressed-on decontamination device (7) with a first contact line (C1), which delimits an emission surface (E) on the decontamination device (7); and
eb) a second circumferential sealing surface (62), which rests against the temporarily pressed-on package (9) with a second contact line (C2), which delimits a process surface (P) on the cover (98) closing the package (9); and
f) the emission surface (E) is at least as large as or larger than the process surface (P), and
that the profile seal (6) has a flank contour (69) facing the spatial content (8), along which:
g) the first contact line (C1), which is intended for interaction with the decontamination device (7) that is temporarily pressed on, lies on the first sealing surface (61);
h) the second contact line (C2) lies on the second sealing surface (62) and is intended for interaction with the cover (98) which closes the temporarily pressed-on package (9); and
i) intermediate lines (Ln) are defined between the first and second contact lines (C1, C2), wherein:
j) the first contact line (C1) has the greatest span (s1) relative to a geometric center line (M) drawn through the cross-section of the profile seal (6), the spans (sn) successively decrease over the intermediate lines (Ln) down to the second contact line (C2) with the smallest span (s2).

2. Sluice arrangement (1) according to claim 1, **characterized in that**, in the projection, the process surface (P) is congruent with the emission surface (E) or the process surface (P) is completely embedded in the larger emission surface (E).

3. Sluice arrangement (1) according to claim 1, **characterized in that**, in the projection, the emission surface (E) is dimensioned to be larger than the process surface (P) and, in this case, the process surface (P) partially projects beyond the emission surface (E).

4. Sluice arrangement (1) according to at least one of claims 1 to 3, **characterized in that** the surfaces enclosing the spatial content (8) comprise at least the emission surface (E), the process surface (P) and the exposed surfaces of the profile seal (6) facing the spatial content (8).

5. Sluice arrangement (1) according to at least one of claims 1 to 4, **characterized in that** a region of the end section (400) of the partition wall (40) can also belong to the surfaces which enclose the spatial content (8) and are to be decontaminated.

6. Sluice arrangement (1) according to at least one of claims 1 to 5, **characterized in that** the profile seal (6) is arranged in one or more parts on that end part (400) of the partition wall (40) which projects into the passage (47).

7. Sluice arrangement (1) according to at least one of claims 1 to 6, **characterized in that** the sealing surfaces (61, 62) are formed at ends of lip-like wings which extend in opposite directions from a base web (60).

8. Sluice arrangement (1) according to claim 7, **characterized in that** the profile seal (6) furthermore comprises:
a) two legs (63, 64) extending from the base web (60), which legs, together with the base web (60), form a channel (65) intended to accommodate the end section (400) of the partition wall (40);
b) a taper (67, 68) at each transition from the respective leg (63, 64) to the neighboring wing, in order to promote material deformation and sealing properties when pressing the decontamination device (7) or the package (9) onto the profile seal (6); and
c) webs (66) directed from the legs (63, 64) into the channel (65) for form-fitting and force-fitting with grooves (401) present in the end section (400).

9. Sluice arrangement (1) according to at least one of claims 1 to 8, **characterized in that** the decontamination device (7) is designed as a radiation source, e.g. UVC, UV, E-Beam or pulsed light.

10. Sluice arrangement (1) according to at least one of claims 1 to 9, **characterized in that** the profile seal (6) consists of an elastomer.

11. Sluice arrangement (1) according to at least one of claims 1 to 10, **characterized in that** the passage (47) is of rectangular or round shape.

12. Sluice arrangement (1) according to at least one of claims 1 to 11, **characterized in that** the container (94) of the package (9) has at the very top a circumferential rim (941), on which the cover (98) spanning the passage (95) is fastened, for example by gluing or welding.

## Revendications

1. Dispositif de sas (1) pour le transfert d'articles (960) dans des conditions aseptiques entre des contenants (9) acheminés en série et une chambre de traitement (41) entourée d'un caisson (49) dans une enceinte de confinement (4) placée dans un local d'installation (R), dans lequel
a) la chambre de traitement (41) est précédée d'un poste d'entrée (3) ;
b) un contenant (9) unique contient :
ba) un récipient (94) avec un espace intérieur (940) aseptique et un passage (95) qui est fermé avec une couverture (98) et
bb) les articles (960) stockés dans l'espace intérieur (940), qui doivent être traités après l'ouverture de la couverture (98) dans la chambre de traitement (41) ;
c) il est prévu pour le transfert des articles (960) des contenants (9) à la chambre de traitement (41) un passage (47) menant du poste d'entrée (3) à la chambre de traitement (41), qui se présente comme une découpe ménagée dans une cloison (40) séparant le poste d'entrée (3) et la chambre de traitement (41) ;
d) le passage (47) est bordé par un joint d'étanchéité profilé (6) qui crée, avec un contenant (9) amené dans le poste d'entrée (3) et pressé temporairement dessus et avec un dispositif de décontamination (7) pressé temporairement dessus, des étanchéités grâce aux quelles les surfaces limitrophes renferment un contenu de l'espace (8) et celui-ci peut être décontaminé avec les surfaces limitrophes au moyen du dispositif de décontamination (7),
e) le joint d'étanchéité profilé (6) possède :
ea) une première surface d'étanchéité circonférentielle (61), qui s'appuie sur le dispositif de décontamination (7) pressé temporairement sur une première ligne de contact (C1) délimitant une surface d'émission (E) sur le dispositif de décontamination (7) et
eb) une deuxième surface d'étanchéité circonférentielle (62) qui s'appuie sur le contenant (9) pressé temporairement sur une deuxième ligne de contact (C2) délimitant une surface de traitement (P) sur la couverture (98) fermant le contenant (9), et
f) la surface d'émission (E) est au moins aussi grande ou plus grande que la surface de traitement (P), et le joint d'étanchéité profilé (6) présente un contour de flancs (69) tourné vers l'intérieur de l'espace (8), le long duquel :
g) la première ligne de contact (C1) destinée à coopérer avec le dispositif de décontamination (7) pressé temporairement s'appuie sur la première surface d'étanchéité (61) ;
h) la deuxième ligne de contact (C2) destinée à coopérer avec la couverture (98) qui ferme le contenant (9) pressé temporairement s'appuie sur la deuxième surface d'étanchéité (62) et
i) des lignes intermédiaires (Ln) sont définies entre la première ligne de contact et la deuxième (C1, C2),
j) la première ligne de contact (C1) présente, par rapport à une ligne médiane géométrique (M) passant par la section du joint d'étanchéité profilé (6), la plus grande portée (s1), les portées (sn) diminuant successivement sur les lignes intermédiaires (Ln) en descendant vers la deuxième ligne de contact (C2) ayant la plus petite portée (s2).

2. Dispositif de sas (1) selon la revendication 1, **caractérisé en ce que**, en projection, la surface de traitement (P) couvre la même aire que la surface d'émission (E) ou la surface de traitement (P) est complètement incluse dans la surface d'émission (E) plus étendue.

3. Dispositif de sas (1) selon la revendication 1, **caractérisé en ce que**, en projection, la surface d'émission (E) a de plus grandes dimensions que la surface de traitement (P) et la surface de traitement (P) chevauche ainsi partiellement la surface d'émission (E).

4. Dispositif de sas (1) selon une au moins des revendications 1 à 3, **caractérisé en ce que** les surfaces renfermant l'intérieur de l'espace (8) comprennent au moins la surface d'émission (E), la surface de traitement (P) et les surfaces libres du joint d'étanchéité profilé (6) tournées vers l'intérieur de l'espace (8).

5. Dispositif de sas (1) selon une au moins des revendications 1 à 4, **caractérisé en ce qu'**une zone de la partie frontale (400) de la cloison (40) peut aussi faire partie des surfaces renfermant l'intérieur de l'espace (8) et à décontaminer.

6. Dispositif de sas (1) selon une au moins des revendications 1 à 5, **caractérisé en ce que** le joint d'étanchéité profilé (6) est disposé en une ou plusieurs parties sur la partie frontale (400) de la cloison (40) qui dépasse dans le passage (47).

7. Dispositif de sas (1) selon une au moins des revendications 1 à 6, **caractérisé en ce que** les surfaces d'étanchéité (61, 62) sont formées aux extrémités d'ailes en forme de lèvres qui s'étendent dans des sens opposés à partir d'une barrette de base (60).

8. Dispositif de sas (1) selon la revendication 7, **caractérisé en ce que** le joint d'étanchéité profilé (6) comporte en outre :
a) deux bras (63, 64) qui s'étendent à partir de la barrette de base (60) et forment avec la barrette de base (60) un canal (65) destiné à recevoir la partie frontale (400) de la cloison (40) ;
b) un resserrement (67, 68) respectif à la transition entre chaque bras (63, 64) et l'aile voisine, visant à favoriser la déformation du matériau et la qualité de l'étanchéité quand le dispositif de décontamination (7) ou le contenant (9) est pressé sur le joint d'étanchéité profilé (6) ; et
c) des barrettes (66) orientées des bras (63, 64) vers le canal (65) pour l'engagement positif et par friction avec des gorges (401) présentes dans la partie frontale (400).

9. Dispositif de sas (1) selon une au moins des revendications 1 à 8, **caractérisé en ce que** le dispositif de décontamination (7) est conçu comme une source de rayonnement, par exemple d'UV-C, d'UV, de faisceau d'électrons ou de lumière pulsée.

10. Dispositif de sas (1) selon une au moins des revendications 1 à 9, **caractérisé en ce que** le joint d'étanchéité profilé (6) se compose d'un élastomère.

11. Dispositif de sas (1) selon une au moins des revendications 1 à 10, **caractérisé en ce que** le passage (47) a une forme rectangulaire ou arrondie.

12. Dispositif de sas (1) selon une au moins des revendications 1 à 11, **caractérisé en ce que** le récipient (94) du contenant (9) possède à son sommet un bord circonférentiel (941) sur lequel la couverture (98) couvrant le passage (95) est fixée, par exemple par collage ou par soudage.
